(19) Europäisches Patentamt
European Patent Office
Office européen des brevets

(11) Veröffentlichungsnummer: **0 039 890**
**B1**

(12) # EUROPÄISCHE PATENTSCHRIFT

(45) Veröffentlichungstag der Patentschrift: **18.05.83**

(51) Int. Cl.³: **C 07 C 43/295**, C 07 C 41/16

(21) Anmeldenummer: **81103410.7**

(22) Anmeldetag: **06.05.81**

(54) **Verfahren zur Herstellung von Hydrochinonmonophenyläthern.**

(30) Priorität: **10.05.80 DE 3018004**

(43) Veröffentlichungstag der Anmeldung: **18.11.81 Patentblatt 81/46**

(45) Bekanntmachung des Hinweises auf die Patenterteilung: **18.05.83 Patentblatt 83/20**

(84) Benannte Vertragsstaaten: **AT BE CH DE FR GB IT LI NL**

(56) Entgegenhaltungen:
**EP-A-0 003 562**
**EP-A-0 008 734**
**GB-A-2 006 211**

**CHEMICAL ABSTRACTS, Band 91, Nr. 15, Oktober 1979, Seite 592, Nr. 123549x Columbus, Ohio, U.S.A.**

(73) Patentinhaber: **HOECHST AKTIENGESELLSCHAFT, Postfach 80 03 20, D-6230 Frankfurt/Main 80 (DE)**

(72) Erfinder: **Geuss, Reinhart, Dr., Völklinger Weg 66, D-6000 Frankfurt am Main 71 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Hydrochinonmonophenyläthern

Hydrochinon-mono-phenyläther der allgemeinen Formel

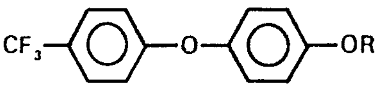

I

in denen R Wasserstoff oder ein einwertiges Kation bedeuten, sind wertvolle Vorprodukte zur Herstellung von selektiven Grasherbiziden (vgl. DOS 24 33 067). Sie werden nach DE-OS 24 33 066 in der Weise erhalten, dass man in an sich bekannter Weise Halogenverbindungen der Formel

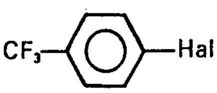

II

in der Hal Halogen bedeutet, mit Hydrochinonderivaten der allgemeinen Formel

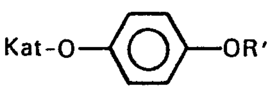

V

umsetzt. Hierbei ist Kat ein einwertiges Kation, R′ kann ausser den Bedeutungen von R auch noch ($C_1$-$C_4$)-Alkyl bedeuten.

Die Umsetzung erfolgt in einem polaren, aprotischen Lösungsmittel bei Reaktionstemperaturen von 120° bis 200°C, vorzugsweise um 170°. Bei R′ = ($C'_1$-$C_4$)Alkyl wird aus dem Reaktionsprodukt die Alkylgruppe durch Ätherspaltung eliminiert.

Ein wesentlicher Nachteil des genannten Verfahrens besteht nun darin, dass, wenn R bzw. R′ ein einwertiges Kation ist, das Endprodukt I mit noch nicht umgesetzten II teilweise zum Hydrochinon-bis-phenyl-äther IV («Bisäther») weiterreagiert:

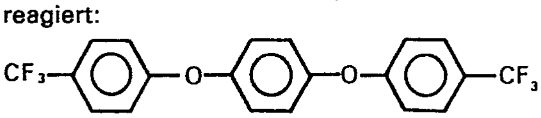

IV

Dies vermindert nicht nur die Ausbeute an I, sondern macht auch ein aufwendiges Aufarbeitungsverfahren für I erforderlich.

In der US-PS 4 172 959 ist beschrieben, dass Bis-äther der Formel IV durch Umsetzung mit Hydrochinon Derivaten der Formel V bei Temperaturen von 120–200° (vorzugsweise ebenfalls 170°) zu Hydrochinon-monophenyläthern spalten kann. Bei der Umsetzung von II mit V (R′=R) konkurrieren also zwei Reaktionen miteinander, die den Gehalt des Bisäthers IV im Reaktionsgemisch bestimmen:

a) I (R=einwertiges Kation)
reagiert mit noch nicht umgesetztem II (Bildungsreaktion von IV)

b) IV reagiert mit nicht umgesetztem V (R′=R) (Spaltungsreaktion von IV).

Aus Gründen der Verfahrensökonomie besteht ein Interesse daran, den Gehalt an Bisäther im Reaktionsgemisch so gering zu halten, dass auf seine Abtrennung und Weiterverarbeitung nach US-PS 4 172 959 verzichtet werden kann. Die Grenze liegt bei 1%.

Diese Forderung kann jedoch unter den im Stand der Technik beschriebenen Bedingungen nicht erfüllt werden.

So liefert nach DOS 24 33 066 z.B. die Umsetzung von V (R′=R) mit II in Dimethylsulfoxid (DMSO) nach 4 Stunden bei 170°C das Endprodukt I.

Der Destillationsrückstand enthält dagegen noch 16% Bisäther.

In Übereinstimmung damit führt die Spaltung des Bisäthers mittels Hydrochinon-dikaliumsalz gemäss US-PS 4 172 959 in DMSO nach 8 Stunden bei 170°C nur in 67%iger Ausbeute zum Hydrochinon-monophenyläther I. Wie bei weiteren Untersuchungen gefunden wurde, kann man nach DOS 24 33 066 bei Anwendung eines Überschusses von ca. 15% Hydrochinondikaliumsalz (DMS/, 8 Stunden bei 170°) die Ausbeute an 4-Trifluor-methyl-4′-hydroxy-diphenyläther (I) zwar auf ca. 85–88% steigern und entsprechend die Bisätherausbeute auf 4% senken; ein weiteres Erhitzen führt aber zu starker Zersetzung des Reaktionsgemisches, bevor es zu einer weitergehenden Spaltung des Bisäthers kommt.

Bei Anwendung anderer Lösungsmittel, wie sie in DE-OS 24 33 066 beschrieben sind, kommt es zwar nicht zur Zersetzung, jedoch ist die Reaktionsgeschwindigkeit wesentlich geringer und der Anteil an Bis-äther höher. Beim Arbeiten in N-Methylpyrrolidon bei 180°C (Überschuss an Hydrochinon-dikaliumsalz wie oben) erreicht die Gesamtausbeute (4-Trifluormethyl-4′-hydroxydiphenyläther plus Bisäther) erst nach 7 Stunden ihren Endwert von 92%, wovon nicht weniger als 21% auf den Bisäther entfallen.

Überraschenderweise zeigte sich nun, dass bei Anwendung höherer Temperaturen (230–260°) unter sonst gleichen Bedingungen der Anteil an Bisäther im Reaktionsgemisch auf unter 1% gesenkt werden kann bei gleichzeitiger Steigerung der Ausbeute I von über 20%.

Gegenstand der Erfindung ist daher ein Verfahren zur Herstellung von Hydrochinon-monophenyläthern der Formel

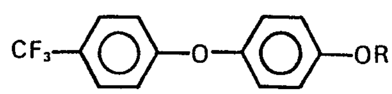

I

worin R Wasserstoff oder ein Alkalikation bedeutet, durch Umsetzung von Halogenverbindungen der Formel

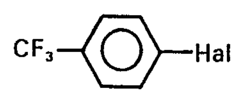

II

mit einem mindestens 10%igen Überschuss eines Hydrochinonderivats der Formel

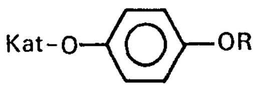

III

in N-Methylpyrrolidon bei Temperaturen zwischen 230°C und 260°C.

Der Überschuss an III beträgt vorzugsweise 15–18%. Grössere Überschüsse als 20% sind zwar unschädlich, führen aber zu einem Mehrverbrauch von Ausgangsmaterial und sind daher unzweckmässig.

Die Reaktionsdauer beträgt etwa 2–10 Stunden. Längere Reaktionszeiten, geringere Überschüsse als 10% und höhere Temperaturen als 260° haben eine Zunahme des Anteils an Bisäther im Reaktionsprodukt zur Folge.

Die Reaktion wird zweckmässigerweise derart durchgeführt, dass zunächst das Ausgangsprodukt III aus Hydrochinon und einem Alkalihydroxid oder -alkoholaten in N-Methylpyrrolidon hergestellt wird.

Man wendet pro Mol Hydrochinon etwa 1,7–2,0 Mol Base (z.B. KOH, NaOH, Natrium- oder Kaliummethylat oder -ethylat) an, ein weiterer Überschuss bringt keinen Vorteil. Der dabei entsprechende Alkohol bzw. das Wasser muss vor der weiteren Umsetzung mit II in üblicher Weise, z.B. durch Destillation, entfernt werden.

Die Lösungsmittelmenge beträgt im allgemeinen zwischen 5 und 10 Gew.-Teile auf 1 Teil der Verbindung III. Die Verbindung II kann bei der Reaktionstemperatur unter Druck zudosiert werden. Einfacher ist es, die Dosierung unterhalb des Siedepunktes des N-Methylpyrrolidons (202°C), also drucklos vorzunehmen. Man wählt dabei zweckmässig die Temperatur so hoch (160–190°C), dass II möglichst rasch zu I bzw. IV reagiert, ohne dass der Rückfluss von II zu stark wird. Dann wird der Reaktionskessel verschlossen und auf die eigentliche Reaktionstemperatur erhitzt.

Zur Aufarbeitung des Reaktionsgemisches destilliert man das Lösungsmittel weitgehend ab, nimmt den Rückstand in Wasser auf, säuert an und extrahiert aus der wässrigen Phase den Hydrochinon-mono-phenyläther mit einem geeigneten Lösungsmittel. Das nach dem Abdampfen des Lösungsmittels verbleibende Rohprodukt kann direkt weiter z.B. nach DE-OS 24 33 067 umgesetzt werden.

Die folgenden Beispiele sollen die Erfindung erläutern, ohne sie zu beschränken.

Beispiel 1

Aus einer Lösung von 4,8 kg (43,6 Mol) Hydrochinon in 60,0 kg N-Methylpyrrolidon und 10,0 kg (89,3 Mol) 50%ige wässrige Kalilauge wird unter Sauerstoffausschluss so lange ein Gemisch N-Methylpyrrolidon/Wasser abdestilliert, bis der Wassergehalt im Destillat kleiner als 1% ist.

In die entstandene Suspension wird bei 175°C innerhalb von 1,5 Std. 6,8 kg (37,7 Mol) p-Chlorbenzotrifluorid eindosiert, das Reaktionsgefäss verschlossen und rasch auf 255°C erhitzt. Der Druck beträgt dabei ca. 2–3 bar. Nach 3 Stunden bei 255°C lässt man abkühlen und destilliert bei 3 Torr das Lösungsmittel weitgehend ab. Den Destillationsrückstand nimmt man in 20 kg Wasser auf, säuert mit konz. Salzsäure an und extrahiert die wässrige Phase mit Octan.

Nach Abdampfen des Octans erhält man 10,4 kg Rohprodukt, das laut Analyse 12,3% N-Methylpyrrolidon, 86,4% 4-Trifluormethyl-4′-hydroxy-diphenyläther (entsprechend 93,7% d.Th.) und 0,5% Hydrochinon-bis-(4-trifluormethyl)phenyläther enthält. Falls erforderlich, kann das Rohprodukt durch Destillation gereinigt werden.

Beispiel 2

Erhitzt man das Reaktionsgemisch nach der Zugabe des p-Chlorbenzotrifluorids 6 Std. auf 235°, so erhält man – nach der oben beschriebenen Aufarbeitung – 10,3 kg Rohprodukt, das 13,0% N-Methylpyrrolidon, 85,3% 4-Trifluormethyl-4′-hydroxydiphenyläther (entsprechend 91,5% d.Th.) und 0,8% Hydrochinon-bis-(4-trifluormethyl)phenyläther enthält.

Beispiel 3

Setzt man an Stelle von 50%iger wässriger Kalilauge 6,3 kg (90,0 Mol) Kaliummethylat ein und destilliert anschliessend das Methanol ab, so erhält man nach dem in Beispiel 1 beschriebenen Verfahren 10,1 kg Rohprodukt, das 12,5% N-Methylpyrrolidon, 84,7% 4-Trifluormethyl-4′-hydroxydiphenyläther (entsprechend 89,3% d.Th.) und 0,6% Hydrochinon-bis-(4-trifluormethyl)phenyläther enthält.

**Patentansprüche**

1. Verfahren zur Herstellung von Hydrochinon-mono-phenyläthern bzw. ihrer Alkalisalze der allgemeinen Formel

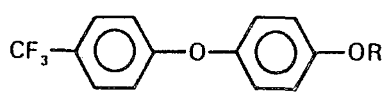

I

in der R Wasserstoff oder ein Alkalikation bedeutet, durch Umsetzung von Halogenverbindungen der Formel

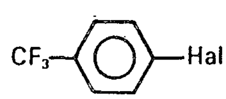

II

in der Hal Chlor oder Brom bedeutet, mit einem mindestens 10%igen Überschuss eines Hydrochinonderivats der Formel

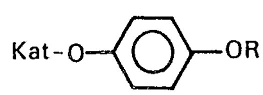

III

in der Kat Natrium oder Kalium bedeutet,

in N-Methylpyrrolidon, dadurch gekennzeichnet, dass man die Umsetzung bei Temperaturen zwischen 230 und 260°C durchführt.

2. Verfahren nach Anspruch 1, dadurch gekennzeichnet, dass der Überschuss an III bezogen auf II 15–18% beträgt.

**Claims**

1. A process for the manufacture of hydroquinone-monophenyl ethers or their alkali metal salts of the general formula

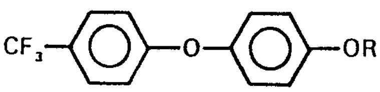

I

in which R is hydrogen or an alkali metal cation by reacting halogen compounds of the formula

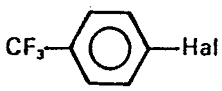

II

in which Hal is chlorine or bromine, with an at least 10% excess of a hydroquinone derivative of the formula

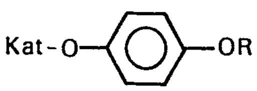

III

in which Kat is sodium or potassium, in N-methyl pyrrolidone, which comprises carrying out the reaction at a temperature between 230 and 260°C.

2. The process as claimed in claim 1, wherein the excess of III relative to II is 15–18%.

**Revendications**

1. Procédé de préparation d'éther monophényliques de l'hydroquinone ou de leurs sels de métaux alcalins, qui répondent à la formule générale

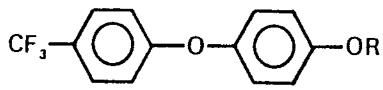

I

dans laquelle R représente l'hydrogène ou un cation de métal alcalin, par réaction de composés halogénés répondant à la formule

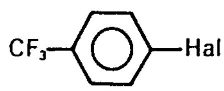

II

dans laquelle Hal représente le chlore ou le brome, avec un excès d'au moins 10% d'un dérivé de l'hydroquinone répondant à la formule

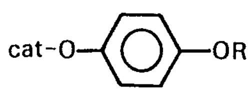

III

dans laquelle Cat représente le sodium ou le potassium, dans de la N-méthylpyrrolidone, procédé caractérisé en ce qu'on effectue la réaction à des températures comprises entre 230 et 260°C.

2. Procédé selon la revendication 1, caractérisé en ce que l'excès du composé (III) par rapport au composé (II) est de 15 à 18%.